# EUROPEAN PATENT SPECIFICATION

(11) **EP 1 989 161 B1**
(45) Date of publication and mention of the grant of the patent: **15.05.2013**
(21) Application number: 07763341.0
(22) Date of filing: 30.01.2007
(51) Int. Cl.: C07C 27/10, C07C 51/16, C07C 67/055, C07C 51/25, C07C 51/215, B01J 23/00

(54) **INTEGRATED PROCESS FOR THE PRODUCTION OF ACETIC ACID AND VINYL ACETATE**
INTEGRIERTES VERFAHREN ZUR HERSTELLUNG VON ESSIGSÄURE UND VINYLACETAT
PROCÉDÉ INTÉGRÉ POUR LA PRODUCTION D'ACIDE ACÉTIQUE ET D'ACÉTATE DE VINYLE

(30) Priority: 07.02.2006 US 765985 P
(43) Date of publication of application: 12.11.2008
(73) Proprietor: Celanese International Corporation, Dallas, TX 75234 (US)
(72) Inventor: MCSWAIN, C., V., Corpus Christi, TX 78413 (US); RYAN, Debra, Ann, League City, TX 77573 (US)
(74) Representative: Kador & Partner
(86) International application number: PCT/US2007/002349
(87) International publication number: WO 2007/092188

(56) References cited:
- WO-A1-2006/055183
- US-A- 2 610 704
- US-A- 5 162 578
- US-A1- 2002 028 965
- US-A1- 2005 020 847
- US-B1- 6 790 983
- US-B1- 7 189 377

## Description

### FIELD OF THE INVENTION

The invention relates generally to an integrated process for the production of acetic acid and vinyl acetate, and in particular to an integrated process for the production of acetic acid and vinyl acetate from a gaseous feedstock comprising essentially ethane.

### BACKGROUND OF THE INVENTION

The oxidative dehydrogenation of ethane to ethylene in the gas phase at temperatures above 500°C has been discussed, for example, in U.S. Patent Nos. 4,250,346, 4,524,236, and 4,568,790.

U.S. Patent No. 4,250,346 describes the use of a catalyst composition containing the elements molybdenum, X and Y in the ratio a:b:c for oxidation of ethane to ethylene, where X is Cr, Mn, Nb, Ta, Ti, V and/or W, and Y is Bi, Ce, Co, Cu, Fe, K, Mg, Ni, P, Pb, Sb, Si, Sn, Tl and/or U, and a is 1, b is from 0.05 to 1, and c is from 0 to 2. The total value of c for Co, Ni and/or Fe must be less than 0.5. The reaction is carried out in the gas phase at temperature below about 550°C. The efficiency of the conversion to ethylene ranges from 50 to 94 percent, depending upon ethane conversion. The catalysts disclosed can likewise be used for the oxidation of ethane to acetic acid, the efficiency of the conversion to acetic acid being about 18 percent, with an ethane conversion of 7.5 percent. Reaction pressures are very low, generally 1 atm, which restricts productivity and commercial viability.

U.S. Patent No. 4,568,790 describes a process for oxidizing ethane to ethylene using an oxide catalyst containing Mo, V, Nb, and Sb. The reaction is preferably carried out at about 200°C to about 450°C. The calculated selectivity for ethylene at 50 percent conversion of ethane ranges from 63 to 76 percent. Again low reaction pressures limit usefulness.

U.S. Patent No. 4,524,236 describes a process for oxidizing ethane to ethylene using an oxide catalyst containing Mo, V, Nb, and Sb and at least one metal from the group consisting of Li, Sc, Na, Be, Mg, Ca, Sr, Ba, Ti, Zr, Hf, Y, Ta, Cr, Fe, Co, Ni, Ce, La, Zn, Cd, Hg, Al, Tl, Pb, As, Bi, Te, U, and W. The reaction is preferably carried out at 200°C to about 400°C. The selectivity for ethylene at 51 percent conversion of ethane is as high as 80 percent for one of the compositions discussed in the '236 patent, but productivity is low.

The above-mentioned specifications are principally concerned with the preparation of ethylene. The use of mixed metal oxide catalysts to convert ethane to acetic acid is also known. For example, U.S. Patent No. 5,162,578 describes a process for the selective preparation of acetic acid from ethane, ethylene or mixtures thereof with oxygen in the presence of a catalyst mixture which comprises at least: (A) a calcined catalyst of the formula MoₓV_{y} or MoₓV_{y}Z_{y}, in which Z can be one or more of the metals Li, Na, Be, Mg, Ca, Sr, Ba, Zn, Cd, Hg, Sc, Y, La, Ce, Al, Tl, Ti, Zr, Hf, Pb, Nb, Ta, As, Sb, Bi, Cr, W, U, Te, Fe, Co and Ni, and x is from 0.5 to 0.9, y is from 0.1 to 0.4, and z is from 0.001 to 1, and (B) an ethylene hydration catalyst and/or ethylene oxidation catalyst. The second catalyst component B is, in particular, a molecular sieve catalyst or a palladium-containing oxidation catalyst. The catalyst mixture was used to produce acetic acid and ethylene from a feed gas mixture consisting of ethane, oxygen, nitrogen and steam. The acetic selectivity was 34 percent and the ethylene selectivity was 62 percent with an ethane conversion of 4 percent. The high conversion rates of ethane were only achieved with the catalyst mixture described, but not in a single catalyst comprising components A and B.

A further process for the preparation of a product comprising ethylene and/or acetic acid is described in European Patent No. EP 0 407 091 B1. According to this process, ethane and/or ethylene and a gas containing molecular oxygen is brought into contact at elevated temperature with a mixed metal oxide catalyst composition of the general formula AₐX_{b}Y_{c} in which A is Mo_{d}Re_{c}W_{f}, X is Cr, Mn, Nb, Ta, Ti, V and/or W; Y is Bi, Cc, Co, Cu, Fe, K, Mg, Ni, P, Pb, Sb, Si, Sn, Tl and/or U; a is 1; b and c are independently 0 to 2; d+e+f=a, and e is nonzero. The selectivity for acetic acid or ethylene could be adjusted by adjusting the ratio of Mo to Re. The maximum selectivity obtained for acetic acid was 78 percent at 14.3 percent ethane conversion. The highest selectivity for ethylene was 70 percent at 15 percent ethane conversion.

Another process for the catalytic production of ethylene from ethane is found in commonly owned U.S. Provisional Patent Applicant No. 60/686,099. According to this process, it is shown to oxidize ethane to ethylene (and some acetic acid) under relatively mild conditions at high selectivity and excellent space-time yields when using a catalyst having the formula MoₐVᵥTaₓTe_{y}. Preferably a is 1.0; v is 0.01 to 1.0, more preferably 0.1 to 0.5; x is 0.01 to 1.0, more preferably 0.05 to 0.2; and y is 0.01 to 1.0, more preferably 0.1 to 0.5. According to the preferred embodiment, the catalyst has the formula Mo_{1.0}V_{0.3}Ta_{0.1}Te_{0.3}O_{z}, where z depends on the oxidation state of the metals and is the number that renders the catalyst electronically neutral.

The selective preparation of acetic acid by catalytic gas-phase oxidation of ethane and/or ethylene in the presence of a palladium-containing catalyst is also well known, for example in commonly owned U.S. Patent No. 6,399,816. According to this process, a gaseous feed comprising ethane, ethylene, and oxygen are brought into contact with a catalyst comprising the elements Mo, Pd, X, and Y in the gram-atomic ratios a:b:c:d in combination with oxygen: Moₐ Pd_{b} X_{c} Y_{d}. X represents one or more of Cr, Mn, Ta, Ti, V, Te, and W. Y represents one or more of B, Al, Ga, In, Pt, Zn, Cd, Bi, Ce, Co, Rh, Ir, Cu, Ag, Au, Fe, Ru, Os, K, Rb, Cs, Mg, Ca, Sr, Ba, Nb, Zr, Hf, Ni, P, Pb, Sb, Si, Sn, Tl and U. The indices a, b, c, and d represent the gram-atomic ratios of the corresponding elements: a=1, b=0.0001 to 0.01, c=0.4 to 1 and d=0.005 to 1. The space-time yield in the oxidation to acetic acid using this process is >150 kg/hm³. The selectivity of the oxidation reaction of ethane and/or ethylene to acetic acid is especially ≥70 mol percent.

Vinyl acetate is generally prepared commercially by contacting acetic acid and ethylene with molecular oxygen in the presence of a catalyst active for the production of vinyl acetate. Suitably, the catalyst may comprise palladium, an alkali metal acetate promoter and an optional co-promoter (for example, gold or cadmium) on a catalyst support.

Integrated processes for producing vinyl acetate are known in the art. For example, commonly owned U.S. Patent No. 6,852,877, discloses a process for the production of vinyl acetate comprising (1) reacting ethane with oxygen in the presence of a catalyst to produce acetic acid (ethane oxidation), (2) reacting ethane with oxygen in the presence of a catalyst to produce ethylene (ethane oxidative dehydrogenation); (3) reacting the ethylene and acetic acid produced above with oxygen in the presence of a catalyst to produce a vinyl acetate product stream; and (4) separating the vinyl acetate from the product stream from step (3).

Also, commonly owned U.S. Patent No. 6,790,983, discloses a process for the production of vinyl acetate comprising (1) reacting ethane with oxygen in the presence of a catalyst to produce acetic acid and ethylene (ethane oxidation), (2) reacting the ethylene and acetic acid produced above with oxygen in the presence of a catalyst to produce a vinyl acetate product stream; and (4) separating the vinyl acetate from the product stream from step (2).

US 2002/0028965 A1, US 2005/0020847 A1 and US 7,189,337 B1 discloses an integrated process for the production of vinyl acetate and/or acetic acid and an apparatus therefore, in which in a first reaction zone ethylene and/or ethane is reacted to produce a first product stream comprising acetic acid and/or ethylene, in a second reaction zone at least a portion of said first product stream comprising acetic acid and/or ethylene is reacted to a second product stream comprising vinyl acetate water and acetic acid, the said second product stream is separated by distillation into an overhead azeotrope fraction comprising vinyl acetate and water and a base fraction comprising acetic acid and the different products are recovered.

WO 2006/055183 A1 discloses a method of coproducing vinyl acetate and ethyl acetate in which in a first reaction step ethylene, acetic acid and oxygen are reacted to vinyl acetate and at least a minor portion of ethyl acetate, a crude product stream containing vinyl acetate and ethyl acetate from said first reaction step is provided to a distillation tower in which the crude product stream is separated into a vinyl acetate product stream enriched in vinyl acetate with respect to the crude product stream and an acid recycle stream enriched in acetic acid with respect to the crude product stream and a mixed side stream containing vinyl acetate and ethyl acetate, the mixed side stream being enriched in ethyl acetate with respect to the vinyl acetate product stream, and the vinyl acetate in the mixed side stream is hydrogenated to provide an ethylene acetate product stream.

As described in these integrated vinyl acetate process patents, using ethane instead of ethylene as feedstock has a significant advantage because ethane is available in natural gas. In the process of natural gas work-up, several ethane-containing mixtures are obtained, which are usually simply flared off, but which all can be used as carbon feedstock for performing the process of the present invention. A specific advantage for an integrated vinyl acetate process is that, in principal, infrastructures, utilities, and other features can be combined, for example only a single feed gas compressor and off-gas scrubbing system is required whereas separate acetic acid and vinyl acetate processes each require their own feed gas compressor and off-gas scrubbing system. Furthermore, an integrated process reduces intermediate storage requirements as compared to two separate processes. All these advantages lead to reduced capital and operating costs.

Although these integrated processes are useful for the conversion of ethane to vinyl acetate, neither is designed for producing sales grade acetic acid in addition to vinyl acetate. It is therefore an object of the invention to provide an integrated process for co-producing sales grade acetic acid and vinyl acetate in a flexible, integrated process based on ethane feedstock.

### SUMMARY OF THE INVENTION

The present invention provides an integrated process for the co-production of acetic acid and vinyl acetate from an essentially ethane feed, comprising the steps reacting ethane with oxygen in the presence of a first catalyst to produce ethylene and some acetic acid, splitting said ethylene in two portions reacting the first portion of the ethylene with oxygen in the presence of a second catalyst to produce acetic acid, and reacting the second portion of the ethylene and a portion of the acetic acid in the presence of a third catalyst to produce vinyl acetate.

### BRIEF DESCRIPTION OF THE DRAWINGS

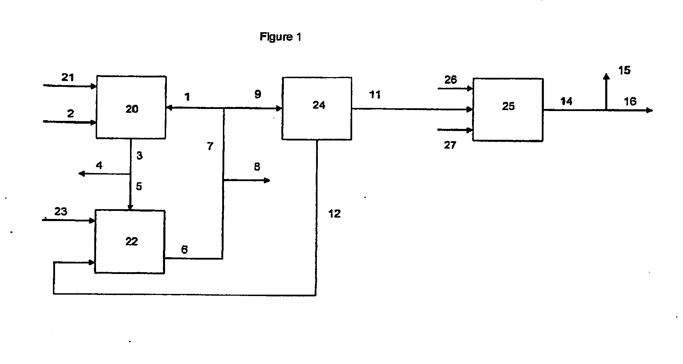

FIGURE 1 shows one embodiment of the ethylene purification process of the present invention.

### DETAILED DESCRIPTION OF THE INVENTION

The present invention provides an integrated process for the co-production of acetic acid and vinyl acetate from an essentially ethane feed, comprising the steps:

(A) reacting ethane with oxygen in the presence of a first catalyst to produce ethylene and some acetic acid;

(B) splitting the ethylene produced in step A into a first portion and a second portion and reacting the first portion of the ethylene from step A with oxygen in the presence of a second catalyst to produce acetic acid; and .

(C) reacting the second portion of the ethylene from step A and a portion of acetic acid from step A or step B in the presence of a third catalyst to produce vinyl acetate.

The process of the present invention will now be illustrated by illustrative example with reference to FIG. 1, which represents a simplified process flow diagram for a preferred embodiment of the present invention.

Reactor 20 contains a catalyst active for the oxidation of ethylene and ethane to acetic acid, as is well known in the art. As described above, a preferred catalyst for Reactor 20 comprises the elements Mo, Pd, X, and Y in the gram-atomic ratios Moₐ Pd_{b} X_{c} Y_{d}. X represents one or more of Cr, Mn, Ta, Ti, V, Te, and W. Y represents one or more of B, Al, Ga, In, Pt, Zn, Cd, Bi, Ce, Co, Rh, Ir, Cu, Ag, Au, Fe, Ru, Os, K, Rb, Cs, Mg, Ca, Sr, Ba, Nb, Zr, Hf, Ni, P, Pb, Sb, Si, Sn, Tl and U. The indices a, b, c, and d represent the gram-atomic ratios of the corresponding elements: a=1, b=0.0001 to 0.01, c=0.4 to 1 and d=0.005 to 1. A preferred catalyst for Reactor 20 is Mo_{1.0} Pd_{0.00075} V_{0.55} Nb_{0.09} Sb_{0.01} Ca_{0.01}.

The reaction of Reactor 20 can be carried out in a fluidized bed or in a fixed-bed reactor. The gaseous feed comprises ethylene and ethane which are fed to the reactor as pure gases or in admixture with one or more other gases. Examples of such additional or carried gases are nitrogen, methane, carbon monoxide, carbon dioxide, air and/or water vapor. The gas comprising molecular oxygen can be air or a gas comprising more or less molecular oxygen than air, e.g. purified oxygen. The ratio of ethane/ethylene to oxygen is advantageously in the range from 1:1 to 10:1, preferably from 2:1 to 8:1. Relatively high oxygen contents are preferred since the achievable ethane conversion and thus the yield of acetic acid is higher.

Although the primary feed for Reactor 20 is a first portion (1) of the ethylene/ethane effluent stream from Reactor 20 (described below), an additional ethane feed stream (2) is preferably added to the Reactor 20 feed. Oxygen feed stream 20 may also be present. This external ethane feed may be substantially pure (i.e. pipeline ethane) or slightly diluted with other gases like the ones generated by natural gas separation (e.g. 90 wt. percent), or may be admixtures with one or more of nitrogen, carbon dioxide, hydrogen, and very low levels of C3/C4 alkenes/alkanes. Catalyst poisons like sulphur and acetylene should be excluded. The amount of inert components is only limited by economics.

The effluent (3) of Reactor 20 comprises primarily acetic acid, water, unreacted ethane, and any unreacted ethylene. An aqueous acetic acid stream (4) is separated from the gas leaving Reactor 20 by condensation, and is further purified through conventional separation technologies such as a drying column and distillation processes to isolate a sales-grade acetic acid product, as is appreciated by one or ordinary skill in the art. The purified acetic acid is then available for sales or for use in a consuming process such as the vinyl acetate Reactor 25 described below. The remaining ethane and unreacted ethylene stream (5) is then fed to Reactor 22, which reacts ethane and oxygen to form ethylene and some acetic acid.

The oxidative dehydrogenation of ethane in Reactor 22 can be carried out in a fluidized bed or in a fixed bed reactor, as is well known to one of ordinary skill in the art. The gaseous feedstock (12) contains primarily ethane but may contain some amount of ethylene, and is fed to the reactor as a pure gas or in a mixture with one or more other gases. Suitable examples of such additional or carrier gases are nitrogen, methane, carbon monoxide, carbon dioxide, air and/or steam. The gas comprising molecular oxygen (23) can be air or a gas comprising more or less molecular oxygen than air, e.g. purified oxygen.

According to a preferred embodiment, the catalyst for Reactor 22 has the formula MoₐVᵥTaₓTe_{y}O_{z}, where a, v, x, y, and z are gram-atomic ratios of the corresponding elements. According to an especially preferred embodiment, the catalyst has the formula Mo_{1.0}V_{0.3}Ta_{0.1}Te_{0.3}O_{z}.

The effluent (6) of Reactor 22 produces a mixture of gases including ethylene, acetic acid, water, COₓ (CO and CO₂), unreacted ethane, and assorted heavy by-products. The product gas effluent from the reactor is preferably filtered to remove catalyst fines and is then routed to a recycle gas scrubber, which produces a top stream (7) containing ethylene, ethane, and COₓ. The bottom stream (8) from the recycle gas scrubber is a crude liquid condensate containing aqueous acetic acid and heavy ends by-products. The crude aqueous acetic acid condensate is purified by conventional separation technologies such as a drying column and distillation to isolate a sales-grade acetic acid product. The purified acetic acid is then available for sales or for use in a consuming process such as the vinyl acetate process described below. -

One of skill in the art will appreciate that the towers, scrubbers, and routing referred to in the preceding paragraphs will have associated with them various heat exchangers, pumps, and connectors and will have operating parameters that are determined by the particular mixture of gases involved. It is within the ability of one of ordinary skill in the art to determine the proper configurations and parameters, given the above disclosure.

The top stream from the recycle gas scrubber containing ethylene, ethane, and COₓ is then split, with a first portion (1) directed as feed to Reactor 1 (as described above), and a second portion (9) directed as a raw feed stream for conversion to vinyl acetate. In a preferred embodiment, the raw feed stream is routed to a fixed bed CO converter followed by a processing step that removes the COₓ from the stream. Carbon dioxide can be removed by any viable technical process known in the art, and for example by reversible absorption in an aqueous K₂CO₃ solution which is then regenerated in a desorption column. The resulting ethylene/ethane stream is then fed to a butane absorption process 24, which purges inert components and concentrates ethylene to greater than 70 percent ethylene, and preferably greater than 75 percent ethylene, by absorbing some of the ethane. With an ethylene feed stream (11) concentration greater than 70 percent ethylene, the Reactor 25 should operate in essentially normal fashion despite the presence of ethane. The absorbed ethane (12) from the butane absorption process is then preferably recycled as feed to Reactor 22. Carbon dioxide is preferably removed from the ethylene feed stream again prior to feeding Reactor 25.

One of ordinary skill in the art will appreciate that Reactor 25 would accommodate any vinyl acetate process known, including preferably vapor phase ethylene acetoxylation utilizing any suitable reactor design capable of removing the heat of reaction in an appropriate way, with the preferred technical solutions being fixed bed or fluidized bed reactors. One process for the vapor phase ethylene acetoxylation reaction of Reactor 25 is described in WO 99/08791, which describes a method for the gas phase oxidation of ethylene and acetic acid to form vinyl acetate. The ethylene reactant used in the present invention is the concentrated ethylene stream (11) as described above. Molecular oxygen-containing gas (26) and purified acetic acid (27) (as described above with respect to Reactor 20 and/or Reactor 22 purified effluents) are added as reactants for Reactor 25 as required per the normal operating conditions of the catalyst and reactor used. One of ordinary skill in the art will appreciate that the ethylene/acetic acid ratio which is necessary for feeding the vinyl acetate reactor may be adjusted as necessary by varying the amount of purified acetic acid added to the feed.

A product stream (14) comprising vinyl acetate, water, ethane, gaseous by-products and unreacted acetic acid and ethylene is withdrawn from Reactor 25 and is typically fed to a scrubber column where a gaseous stream (15) comprising ethylene and ethane together with inerts, carbon monoxide and carbon dioxide by-products is withdrawn overhead. This gas stream may be split (not shown), with a first portion recycled as feed to Reactor 20, and optionally a second portion fed to the butane absorption unit (24). A liquid stream (16) comprising vinyl acetate, water, unreacted acetic acid and possibly other high boiling products of the process are withdrawn from the base of the scrubber column. Vinyl acetate is isolated from this stream by any means known to one of ordinary skill in the art. For example, the liquid stream may be fed to a distillation column where vinyl acetate and water is removed as an azeotrope, with acetic acid and other high boiling products being removed as a bleed from the base of the distillation column. The water in the overhead stream from the distillation column can be separated from the vinyl acetate in a decanter and a vinyl acetate product stream removed from decanter is purified by conventional means known in the art.

## Claims

1. An integrated process for the production of vinyl acetate from an ethane feed, comprising the steps:
(A) reacting ethane with oxygen in the presence of a first catalyst to produce ethylene and acetic acid;
(B) splitting the ethylene produced in step A into a first portion and a second portion and reacting the first portion of the ethylene from step A with oxygen in the presence of a second catalyst to produce acetic acid; and
(C) reacting the second portion of the ethylene from step A and at least a portion of acetic acid from step A or step B in the presence of a third catalyst to produce vinyl acetate.

2. The integrated process of claim 1, wherein the second catalyst has the formula MoₐPd_{b}X_{c}Y_{d};
wherein X is at least one element selected from the group consisting of Cr, Mn, Ta, Ti, V, Te, and W;
wherein Y is at least one element selected from the group consisting of B, Al, Ga, In, Pt, Zn, Cd, Bi, Ce, Co, Rh, Ir, Cu, Ag, Au, Fe, Ru, Os, K, Rb, Cs, Mg, Ca, Sr, Ba, Nb, Zr, Hf, Ni, P, Pb, Sb, Si, Sn, Tl and U; and
wherein a, b, c, and d are gram-atomic ratios and denote a = 1, b = 0.0001-0.01, c = 0.4-1 and d = 0.005-1.

3. The integrated process of claim 1, wherein the second catalyst has the formula Mo_{1.0}Pd_{0.00075}V_{0.55} Nb_{0.09}Sb_{0.01}Ca_{0.01}.

4. The integrated process of claim 1, wherein the first catalyst has the formula MoₐVᵥTaₓTe_{y};
wherein a, v, x, and y are gram-atomic ratios and denote a = 1.0; v = 0.01-1.0, x = 0.01- 1.0, and y =0.01-1.0.

5. The integrated process of claim 1, wherein the first catalyst for step (A) has the formula Mo_{1.0}V_{0.3}Ta_{0.1}Te_{0.3}.

6. The process of claim 1, wherein step (C) is performed using a vapor phase ethylene acetoxylation reaction.

7. The process of claim 6, wherein the reaction further comprises the addition of molecular oxygen containing gas and purified acetic acid.

8. The process of claim 1, further comprising exposing the first portion to a scrubber column.

9. The process of claim 1, wherein step (A) has a ratio of ethane/ethylene to oxygen is 1:1 to 10:1.

10. The process of claim 1, wherein step (A) further comprises exposing the ethylene and acetic acid to a condenser.

11. The process of claim 1, wherein step (B) further comprises exposing the acetic acid to a scrubber.

12. The process of claim 1, wherein step (B) further comprises flowing the acetic acid through a COₓ converter.

13. The process of claim 1, wherein step (B) further comprises flowing the ethylene through a COₓ converter before reacting a first portion of the ethylene.

14. The process of claim 1, wherein step (C) further comprises flowing the ethylene through a COₓ converter before reacting a second portion of the ethylene.

15. The process of claim 1, wherein step (A) further comprises exposing the ethane to a distillation column.

16. The process of claim 1, wherein step (B) has a ratio of ethane/ethylene to oxygen that is 1:1 to 10:1.

## Patentansprüche

1. Integriertes Verfahren zur Herstellung von Vinylacetat aus einer Ethanbeschickung, das die folgenden Schritte aufweist:
(A) Umsetzen von Ethan mit Sauerstoff in Gegenwart eines ersten Katalysators, um Ethylen und Essigsäure zu erzeugen;
(B) Aufteilen des im Schritt A erzeugten Ethylens in einen ersten Anteil und einen zweiten Anteil und Umsetzen des ersten Anteils des Ethylens vom Schritt A mit Sauerstoff in Gegenwart eines zweiten Katalysators, um Essigsäure zu erzeugen; und
(C) Umsetzen des zweiten Anteils des Ethylens vom Schritt A und von zumindest einem Teil der Essigsäure vom Schritt A oder vom Schritt B in Gegenwart eines dritten Katalysators, um Vinylacetat zu erzeugen.

2. Integriertes Verfahren nach Anspruch 1, wobei der zweite Katalysator die Formel MoₐPd_{b}X_{c}Y_{d} hat;
wobei X zumindest ein Element ist, das aus der Gruppe ausgewählt ist, bestehend aus Cr, Mn, Ta, Ti, V, Te und W;
wobei Y zumindest ein Element ist, das aus der Gruppe ausgewählt ist, bestehend aus B, Al, Ga, In, Pt, Zn, Cd, Bi, Ce, Co, Rh, Ir, Cu, Ag, Au, Fe, Ru, Os, K, Rb, Cs, Mg, Ca, Sr, Ba, Nb, Zr, Hf, Ni, P, Pb, Sb, Si, Sn, Tl und U; und
wobei a, b, c und d auf Grammatom bezogene Verhältnisse sind und folgendes bedeuten: a = 1, b = 0,0001 bis 0,01, c = 0,4 bis 1 und d = 0,005 bis 1.

3. Integriertes Verfahren nach Anspruch 1, wobei der zweite Katalysator die Formel Mo_{1,0}Pd_{0,00075}V_{0,55}Nb_{0,09}Sb_{0,01}Ca_{0,01} hat.

4. Integriertes Verfahren nach Anspruch 1, wobei der erste Katalysator die Formel MoₐVᵥTaₓTe_{y} hat;
wobei a, v, x und y auf Grammatom bezogene Verhältnisse sind und folgendes bedeuten: a = 1,0, v = 0,01 bis 1,0, x = 0,01 bis 1,0 und y = 0,01 bis 1,0.

5. Integriertes Verfahren nach Anspruch 1, wobei der erste Katalysator für den Schritt (A) die Formel Mo_{1,0}V_{0,3}Ta_{0,}1Te_{0,3} hat.

6. Verfahren nach Anspruch 1, wobei der Schritt (C) unter Anwendung einer Acetoxylierungsreaktion von Ethylen in der Dampfphase erfolgt.

7. Verfahren nach Anspruch 6, wobei die Reaktion ferner die Zugabe von molekularen Sauerstoff enthaltendem Gas und gereinigter Essigsäure aufweist.

8. Verfahren nach Anspruch 1, das ferner das Aussetzen des ersten Anteils einer Wäscherkolonne aufweist.

9. Verfahren nach Anspruch 1, wobei der Schritt (A) ein Verhältnis von Ethan/Ethylen zu Sauerstoff aufweist, das 1:1 1 bis 10:1 beträgt.

10. Verfahren nach Anspruch 1, wobei der Schritt (A) ferner das Aussetzen von Ethylen und Essigsäure einem Kondensator aufweist.

11. Verfahren nach Anspruch 1, wobei der Schritt (B) ferner das Aussetzen der Essigsäure einem Gaswäscher aufweist.

12. Verfahren nach Anspruch 1, wobei der Schritt (B) ferner das Strömen der Essigsäure durch einen CO_{X}-Konverter aufweist.

13. Verfahren nach Anspruch 1, wobei der Schritt (B) ferner das Strömen des Ethylens durch einen CO_{X}-Konverter umfaßt, bevor der erste Anteil des Ethylens umgesetzt wird.

14. Verfahren nach Anspruch 1, wobei der Schritt (C) ferner das Strömen des Ethylens durch einen CO_{X}-Konverter aufweist, bevor ein zweiter Anteil des Ethylens umgesetzt wird.

15. Verfahren nach Anspruch 1, wobei der Schritt (A) ferner das Aussetzen des Ethans einer Destillationskolonne aufweist.

16. Verfahren nach Anspruch 1, wobei der Schritt (B) ein Verhältnis von Ethan/Ethylen zu Sauerstoff aufweist, das 1:1 bis 10:1 beträgt.

## Revendications

1. Procédé intégré de production d'acétate de vinyle à partir d'une matière première d'éthane comprenant les étapes de :
(A) réaction de l'éthane avec de l'oxygène en présence d'un premier catalyseur pour produire de l'éthylène et de l'acide acétique ;
(B) séparation de l'éthylène produit dans l'étape A en une première partie et une seconde partie et réaction de la première partie de l'éthylène de l'étape A avec de l'oxygène en présence d'un second catalyseur pour produire de l'acide acétique ; et
(C) réaction de la seconde partie de l'éthylène de l'étape A et d'au moins une partie de l'acide acétique de l'étape A ou l'étape B en présence d'un troisième catalyseur pour produire de l'acétate de vinyle.

2. Procédé intégré de la revendication 1, dans lequel le second catalyseur a la formule MoₐPd_{b}X_{c}Y_{d} ;
X étant au moins un élément choisi dans le groupe constitué de Cr, Mn, Ta, Ti, V, Te, et W ;
Y étant au moins un élément choisi dans le groupe constitué de B, Al, Ga, In, Pt, Zn, Cd, Bi, Ce, Co, Rh, Ir, Cu, Ag, Au, Fe, Ru, Os, K, Rb, Cs, Mg, Ca, Sr, Ba, Nb, Zr, Hf, Ni, P, Pb, Sb, Si, Sn, Tl et U ; et
a, b, c, et d étant des rapports atomiques en gramme et désignant a = 1, b = 0,0001 à 0,01, c = 0,4 à 1 et d = 0,005 à 1.

3. Procédé intégré de la revendication 1, dans lequel le second catalyseur a la formule Mo_{1,0}Pd_{0,00075}Y_{0,55}Nb_{0,09}Sb_{0,01}Ca_{0,01}.

4. Procédé intégré de la revendication 1, dans lequel le premier catalyseur a la formule MoₐVᵥTaₓTe_{y} ;
a, v, x et y étant des rapports atomiques en gramme et désignant a = 1,0 ;
v = 0,01 à 1,0, x = 0,01 à 1,0, et y = 0,01 à 1,0.

5. Procédé intégré de la revendication 1, dans lequel le premier catalyseur pour l'étape (A) a la formule Mo_{1,0}V_{0,3}Ta_{0,1}Te_{0,3}.

6. Procédé de la revendication 1, dans lequel l'étape (C) est effectuée en utilisant une réaction d'acétoxylation de l'éthylène en phase vapeur.

7. Procédé de la revendication 6, dans lequel la réaction comprend en outre l'ajout de gaz contenant de l'oxygène moléculaire et d'acide acétique purifié.

8. Procédé de la revendication 1, comprenant en outre l'exposition de la première partie à une colonne d'épuration.

9. Procédé de la revendication 1, dans lequel l'étape (A) a un rapport de l'éthane/éthylène à l'oxygène qui est de 1:1 à 10:1.

10. Procédé de la revendication 1, dans lequel l'étape (A) comprend en outre l'exposition de l'éthylène et de l'acide acétique à un condenseur.

11. Procédé de la revendication 1, dans lequel l'étape (B) comprend en outre l'exposition de l'acide acétique à un épurateur.

12. Procédé de la revendication 1, dans lequel l'étape (B) comprend en outre la circulation de l'acide acétique à travers un convertisseur de COₓ.

13. Procédé de la revendication 1, dans lequel l'étape (B) comprend en outre la circulation de l'éthylène à travers un convertisseur de COₓ avant la réaction d'une première partie de l'éthylène.

14. Procédé de la revendication 1, dans lequel l'étape (C) comprend en outre la circulation de l'éthylène à travers un convertisseur de COₓ avant la réaction d'une seconde partie de l'éthylène.

15. Procédé de la revendication 1, dans lequel l'étape (A) comprend en outre l'exposition de l'éthane à une colonne de distillation.

16. Procédé de la revendication 1, dans lequel l'étape (B) a un rapport de l'éthane/éthylène à l'oxygène qui est de 1:1 à 10:1.
